# EUROPEAN PATENT APPLICATION

(11) **EP 4 850 669 A1**
(43) Date of publication of application: **09.12.2026**
(21) Application number: 25748701.7
(22) Date of filing: 30.01.2025
(51) Int. Cl.: A61K 31/403, A61K 31/496, A61K 31/4045, A61K 31/4196, A61K 31/4425, A61P 25/00, A61P 25/02, A61P 25/14, C07D 209/34

(54) **THERAPEUTIC AGENT AND THERAPEUTIC COMPOSITION FOR HUNTINGTON'S DISEASE**

(30) Priority: 31.01.2024 JP 2024012936; 02.10.2024 JP 2024173883
(71) Applicant: K Pharma, Inc., Tokyo 106-0032 (JP)
(72) Inventor: YOSHIDA Shunsuke, Fujisawa-shi, Kanagawa 251-8555 (JP)
(74) Representative: Müller, Christian Stefan Gerd
(86) International application number: PCT/JP2025/002898
(87) International publication number: WO 2025/164698

(57) **Abstract**

The present invention addresses the problem of providing a therapeutic agent for Huntington's disease, a therapeutic composition for Huntington's disease or a therapeutic method for Huntington's disease, for which no therapeutic agent has been developed so far. The inventors of the present invention induced differentiation of iPS cells derived from a patient with Huntington's disease to striatal medium spiny neurons (MSNs) and found that by administering, to the striatal MSNs reflecting the pathology of Huntington's disease, a therapeutic agent for Huntington's disease containing a compound represented by formula (1-1), a compound represented by formula (2-1), a compound represented by formula (3-1), a compound represented by formula (4-1), a pharmaceutically acceptable salt thereof, or a solvate thereof, the pathology of Huntington's disease in the striatal MSNs was ameliorated.

## Description

### Technical Field

The object of the present invention is to develop a therapeutic agent or a therapeutic composition for Huntington's disease.

### Background Art

Huntington's disease is a hereditary neurodegenerative disease exhibiting autosomal dominant inheritance, characterized by involuntary movements such as chorea, psychiatric symptoms, behavioral abnormalities, and cognitive impairment.

This disease is thought to be caused by an abnormal elongation of the CAG repeat sequence (CAG repeat) present in the huntingtin (HTT) gene (IT15 gene) located on chromosome 4 in patients with Huntington's disease. Since such genetic abnormalities are hereditary and passed on to offspring, it is considered one of the autosomal dominant inheritance diseases. A pathological image has been observed in which the abnormal HTT protein caused by this mutated HTT gene accumulates in cells.

The onset and timing of Huntington's disease are thought to be related to the number of CAG repeats in the HTT gene. Huntington's disease develops when there are 40 or more CAG repeats, and the higher the number of CAG repeats, the more likely it is to develop at a young age.

The incidence of the disease tends to differ slightly depending on race. In Japan, it is reported to be 0.7 per 100,000 people, while in Caucasians it is 4 to 8 per 100,000 people. As of 2021, 918 patients have been reported in Japan, and it is thought that there are about 10 times that number in Europe and the United States.

Huntington's disease is thought to develop when the abnormal HTT protein described above forms aggregates of various structural forms, each exhibiting different cytotoxicities, causing degeneration of nerve cells in the striatum and cerebral cortex.

The symptoms of Huntington's disease progress in the following order: initial symptoms, motor symptoms, involuntary movements, and psychiatric symptoms. The symptoms include:
- Initial symptoms: motor symptoms such as difficulty with fine motor skills, grimacing, and involuntary hand movements; psychiatric symptoms and behavioral abnormalities such as restlessness and depression;
- Motor symptoms: Initially, fine motor skills (such as writing) are often impaired, and the ability to repeat the same action becomes difficult, leading to dropping objects and falling. As the condition progresses, gait becomes unstable, stumbling and falling are more likely, eating becomes difficult, and speech becomes difficult;
- Involuntary movements: Symptoms such as rapid facial and limb movements that occur independently of one's will, irregular and involuntary hand movements, neck movements, grimacing, and clicking of the tongue (known as chorea);
- Psychiatric symptoms: Unlike ordinary dementia, the ability to plan and execute, and the ability to grasp the overall picture tend to be impaired, and personality and behavioral changes such as irritability and excessive repetition of the same things occur.

Currently, symptomatic treatment is used for some symptoms of Huntington's disease, such as involuntary movements like chorea and psychiatric symptoms like depression, personality disorders, and irritability, by administering medications to alleviate these symptoms. For chorea, among the symptoms of Huntington's disease, tetrabenazine, an antipsychotic drug that exhibits dopamine reuptake inhibition (release inhibition), is used.

However, currently, there are no drugs that can be used for a fundamental cure of Huntington's disease (improvement of motor function, psychiatric symptoms, and cognitive function), and a treatment method aimed at a cure has not yet been established. Therefore, the development of medicines or treatment methods that can improve motor function, psychiatric symptoms, and cognitive function is desired.

### Citation List

### Non Patent Literature

[Non-Patent Literature 1] Wu M., et al., A Chemical Recipe for Generation of Clinical-Grade Striatal Neurons from hESCs., Stem cell reports, 11(3), 2018 [Non-Patent Literature 2] Victor MB., et al., Generation of human striatal neurons by microRNA-dependent direct conversion of fibroblasts., Neuron, 84(2), 2015

### Summary of Invention

### Technical Problem

The object of the present invention is to provide a therapeutic agent for Huntington's disease or a pharmaceutical composition for treating Huntington's disease, or a method for treating Huntington's disease, for which no therapeutic drugs have been developed to date.

### Solution to Problem

The inventors of the present invention have found that by differentiating striatal-type medium spiny neurons (MSNs) from iPS cells derived from Huntington's disease patients, and by administering a therapeutic agent for Huntington's disease described later to the striatum medium spiny neurons (MSNs) that reflect the pathology of Huntington's disease, the pathology of Huntington's disease in the striatum medium spiny neurons (MSNs) was improved.

Based on this finding, the present invention has shown that Huntington's disease can be treated with a therapeutic agent for Huntington's disease comprising the compound represented by the formula (1-1), the compound represented by the formula (2-1), the compound represented by the formula (3-1), or the compound represented by the formula (4-1), a pharmaceutically acceptable salt thereof, or a solvate thereof, as described later.

More specifically, the present application provides the following embodiments in order to solve the aforementioned problems:
[1]: A therapeutic agent for Huntington's disease comprising:
   a compound represented by the following formula (1-1): 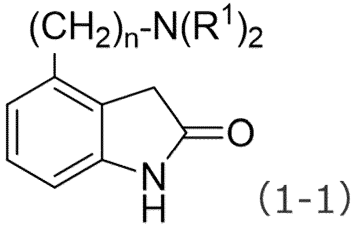 [wherein, in the formula (1-1), R¹ independently represents an alkyl group having 1 to 6 carbon atoms or a 4-hydroxyphenethyl group, and n represents an integer from 1 to 3],
   a compound represented by the following formula (2-1): 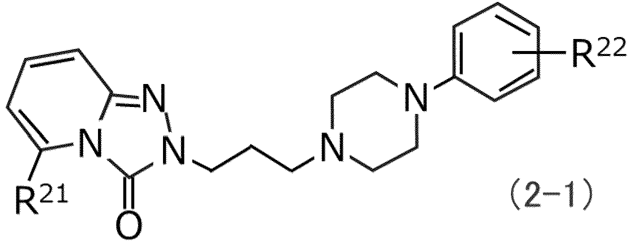
      [wherein, in the formula (2-1), R²¹ is H or -CH₃,
      R²² is selected from the group consisting of H, an alkyl group having 1 to 6 carbon atoms, an alkoxy group having 1 to 6 carbon atoms, and a halogen],
   a compound represented by the following formula (3-1): 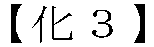 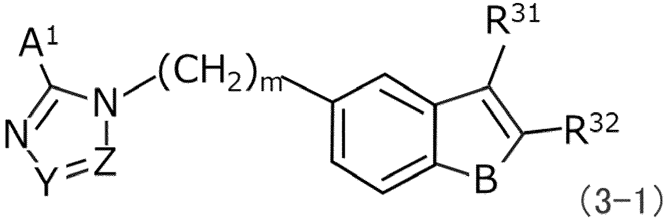
      [wherein, in the formula (3-1),
      Y represents N or A²-C,
      Z represents N or CH,
      m is 0, 1, 2 or 3,
      B represents O, S or N-R³³,
      A¹ and A² independently represent an optionally substituted alkyl group having 1 to 6 carbon atoms, an optionally substituted alkenyl group having 2 to 6 carbon atoms, an optionally substituted alkynyl group having 2 to 6 carbon atoms, an optionally substituted cycloalkyl group having 3 to 7 carbon atoms, an optionally substituted aryl group, an optionally substituted aryl-alkyl group having 1 to 6 carbon atoms, an optionally substituted heterocycloalkyl group having 3 to 7 carbon atoms, an optionally substituted heteroaryl group, an optionally substituted heteroaryl-alkyl group having 1 to 6 carbon atoms, or H, halogen, cyano, trifluoromethyl, an alkoxy group having 1 to 6 carbon atoms, an alkylthio group having 1 to 6 carbon atoms, or -NR^{x}R^{y},
      wherein R^{x} and R^{y} independently represent H, a hydrocarbon group, or a heterocyclic group, or R^{x} and R^{y} together represent an alkylene group having 2 to 6 carbon atoms,
      R31 represents -CH₂CHR34NR35R36 or the following formula: 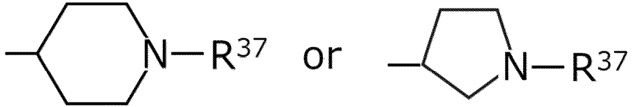
      wherein R³², R³³, R³⁴, R³⁵, R³⁶, and R³⁷ independently represent H or an alkyl group having 1 to 6 carbon atoms],
   a compounds represented by the following formula (4-1): 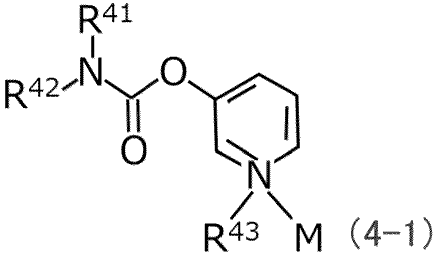
      [wherein, in the formula (4-1),
      R⁴¹ and R⁴² are selected from an alkyl group having 1 to 6 carbon atoms, an aryl group having 6 to 10 carbon atoms, and an alkyl group having 1 to 6 carbon atoms substituted with an aryl group having 6 to 10 carbon atoms, R⁴³ is selected from an alkyl group having 1 to 6 carbon atoms,
      M is selected from halogens and alkyl sulfates]
      or a pharmaceutically acceptable salt thereof, or a solvate thereof.
[2]: The therapeutic agent for Huntington's disease according to [1], wherein n is 2 in the formula (1-1).
[3]: The therapeutic agent for Huntington's disease according to [1] or [2], wherein R¹ in the formula (1-1) is an n-propyl group.
[4]: The therapeutic agent for Huntington's disease according to [1] or [2], wherein the compound represented by the formula (1-1) is a compound represented by the following formula (1-2): 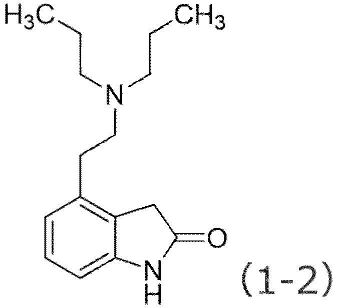 (4-[2-(dipropylamino)ethyl]-1,3-dihydro-2H-indole-2-one).
[5]: The therapeutic agent for Huntington's disease according to [4], wherein the pharmaceutically acceptable salt of the compound represented by the formula (1-1) is a hydrochloride salt of the compound represented by the formula (1-2) (4-[2-(dipropylamino)ethyl]-1,3-dihydro-2H-indole-2-one hydrochloride).
[6]: The therapeutic agent for Huntington's disease according to [1] or [2], wherein the compound represented by the formula (2-1) is a compound represented by the following formula (2-2): 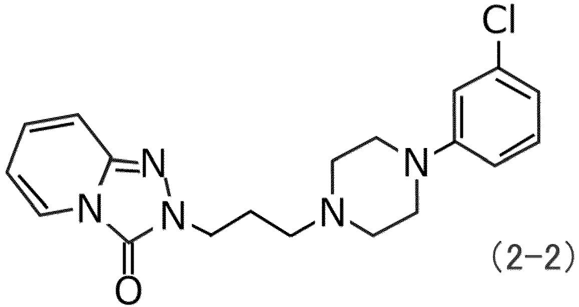 (2-[3-[4-(m-chlorophenyl)-1-piperazinyl]propyl]-s-triazolo[4,3-a]pyridine-3(2H)-one).
[7]: The therapeutic agent for Huntington's disease according to [6], wherein the pharmaceutically acceptable salt of the compound represented by the formula (2-1) is a hydrochloride salt of the compound represented by the formula (2-2) (2-[3-[4-(m-chlorophenyl)-1-piperazinyl]propyl]-s-triazolo[4,3-a]pyridine-3(2H)-one hydrochloride).
[8]: The therapeutic agent for Huntington's disease according to [1] or [2], wherein the compound represented by the formula (3-1) is a compound represented by the following formula (3-2): 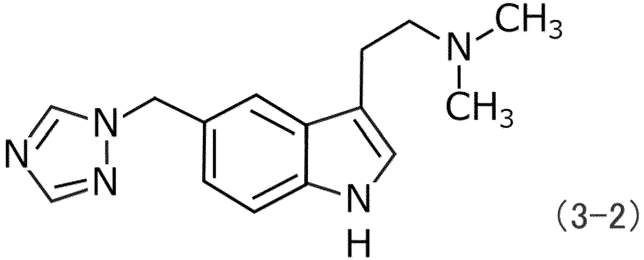 (N,N-dimethyl-2-[5-(1H-1,2,4-triazole-1-ylmethyl)-1H-indole-3-yl]ethaneamine).
[9]: The therapeutic agent for Huntington's disease according to [8], wherein the pharmaceutically acceptable salt of the compound represented by the formula (3-1) is a benzoate of the compound represented by the formula (3-2) (N,N-dimethyl-2-[5-(1H-1,2,4-triazole-1-ylmethyl)-1H-indole-3-yl]ethaneamine benzoate).
[10]: The therapeutic agent for Huntington's disease according to [1] or [2], wherein the compound represented by the formula (4-1) is a compound represented by the following formula (4-2):

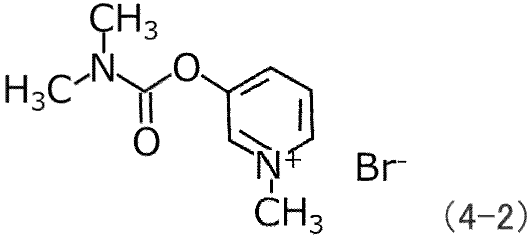

   (3-dimethylcarbamoyloxy-1-methylpyridinium bromide).
[11]: A pharmaceutical composition for treating Huntington's disease comprising:
   a compound represented by the following formula (1-1): 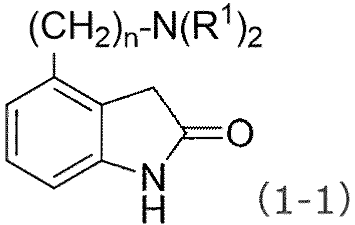 [wherein, in the formula (1-1), R¹ independently represents an alkyl group having 1 to 6 carbon atoms or a 4-hydroxyphenethyl group, and n represents an integer from 1 to 3],
   a compound represented by the following formula (2-1): 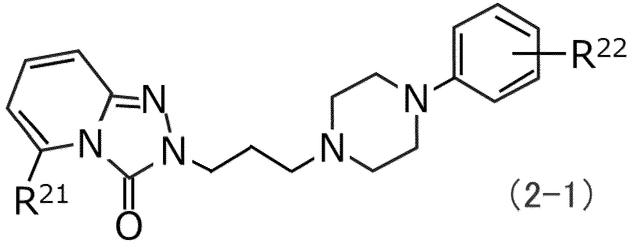
      [wherein, in the formula (2-1), R²¹ is H or -CH₃,
      R²² is selected from the group consisting of H, an alkyl group having 1 to 6 carbon atoms, an alkoxy group having 1 to 6 carbon atoms, and a halogen],
   a compound represented by the following formula (3-1): 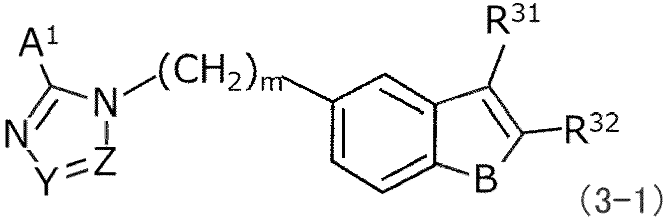
      [wherein, in the formula (3-1),
      Y represents N or A²-C,
      Z represents N or CH,
      m is 0, 1, 2 or 3,
      B represents O, S or N-R³³,
      A1 and A2 independently represent an optionally substituted alkyl group having 1 to 6 carbon atoms, an optionally substituted alkenyl group having 2 to 6 carbon atoms, an optionally substituted alkynyl group having 2 to 6 carbon atoms, an optionally substituted cycloalkyl group having 3 to 7 carbon atoms, an optionally substituted aryl group, an optionally substituted aryl-alkyl group having 1 to 6 carbon atoms, an optionally substituted heterocycloalkyl group having 3 to 7 carbon atoms, an optionally substituted heteroaryl group, or an optionally substituted heteroaryl-alkyl group having 1 to 6 carbon atoms, or H, halogen, cyano, trifluoromethyl, an alkoxy group having 1 to 6 carbon atoms, an alkylthio group having 1 to 6 carbon atoms, or -NR^{x}R^{y},
      wherein R^{x} and R^{y} independently represent H, a hydrocarbon group, or a heterocyclic group, or R^{x} and R^{y} together represent an alkylene group having 2 to 6 carbon atoms,
      R31 represents -CH₂CHR34NR35R36 or the following formula: 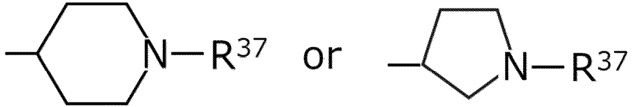
      R³², R³³, R³⁴, R³⁵, R³⁶, and R³⁷ independently represent H or an alkyl group having 1 to 6 carbon atoms],
   a compound represented by the following formula (4-1): 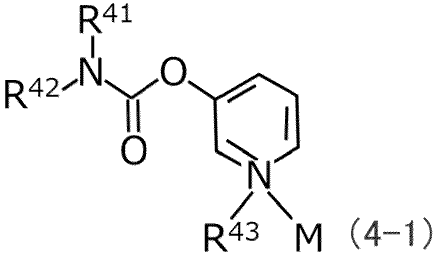
      [wherein in the formula (4-1),
      R⁴¹ and R⁴² are selected from an alkyl group having 1 to 6 carbon atoms, an aryl group having 6 to 10 carbon atoms, and an alkyl group having 1 to 6 carbon atoms substituted with an aryl group having 6 to 10 carbon atoms, R⁴³ is selected from an alkyl group having 1 to 6 carbon atoms,
      M is selected from halogens and alkyl sulfates],
   or a pharmaceutically acceptable salt thereof, or a solvate thereof, as an active ingredient.

### Advantageous Effects of Invention

The therapeutic agent for Huntington's disease of the present invention is a compound obtained by screening a library of compounds with phenotypes characteristic of the human disease state as evaluation items, based on analysis using striatal-type medium spiny neurons (MSNs) differentiated from iPS cells derived from Huntington's disease patients as a disease model. Therefore, the present invention can provide a therapeutic agent with high therapeutic efficacy against Huntington's disease.

### Brief Description of Drawings

[Figure 1] Figure 1 shows a scheme of cell culture for producing striatal-type medium spiny neurons (MSNs).
[Figure 2] Figure 2 shows immunostained cells positive for the MSN markers DARPP32 and DRD2, which were derived from differentiated healthy individuals.
[Figure 3] Figure 3 shows results of examining the percentage (%) of cells positive for the MSN markers DARPP32 and DRD2, by immunostaining differentiated cells.
[Figure 4] Figure 4 shows results of examining LDH leakage, a phenotype of striatal-type MSNs derived from Huntington's disease-specific iPS cells, showing that the LDH leakage rate is significantly higher in the MSN cells derived from disease-specific iPS cells.
[Figure 5] Figure 5 shows results of the phenotype of striatal type MSN derived from Huntington's disease-specific iPS cells, showing that neurite length is significantly reduced in the MSN cells derived from disease-specific iPS cells.
[Figure 6] Figure 6 shows results of examining neurite length, a phenotype of striatal-type MSN derived from Huntington's disease-specific iPS cells, showing that neurite length is significantly reduced over time in the MSN cells derived from disease-specific iPS cells.
[Figure 7] Figure 7 shows results of the phenotype of striatal-type MSN derived from Huntington's disease-specific iPS cells, showing that HTT aggregates are significant accumulated in the MSN cells derived from disease-specific iPS cells.
[Figure 8] Figure 8 shows results of examining the accumulation of HTT aggregates, a phenotype of striatal-type MSN derived from Huntington's disease-specific iPS cells, showing that HTT aggregates are significant accumulated over time in the MSN cells derived from disease-specific iPS cells.
[Figure 9] Figure 9 shows procedure for screening compounds for selecting therapeutic drugs for Huntington's disease using striatal-type MSN derived from Huntington's disease-specific iPS cells.
[Figure 10-1] Figure 10-1 shows disease improvement rate (%) calculated based on three indicators when each of the eight compounds obtained from screening of compounds was applied to cells with 180 CAG repeats (HD1.CAG180).
[Figure 10-2] Figure 10-2 shows disease improvement rate (%) calculated based on three indicators when each of the eight compounds obtained from screening of compounds was applied to cells with 50 CAG repeats (HD2.CAG50).
[Figure 11] Figure 11 shows effects of various concentrations of ropinirole on neurite retraction.
[Figure 12] Figure 12 shows effects of various concentrations of ropinirole on HTT aggregates.
[Figure 13] Figure 13 shows procedure for investigating effects of ropinirole using striatal-type MSNs derived from Huntington's disease-specific iPS cells.
[Figure 14] Figure 14 shows effects of various concentrations of ropinirole on LDH leakage using striatal-type MSNs derived from Huntington's disease-specific iPS cells.
[Figure 15] Figure 15 shows effects of various concentrations of ropinirole on LDH leakage using striatal-type MSNs derived from Huntington's disease-specific iPS cells.
[Figure 16] Figure 16 shows effects of various concentrations of ropinirole on HTT aggregates using striatal-type MSN derived from Huntington's disease-specific iPS cells.
[Figure 17] Figure 17 shows effects of various concentrations of ropinirole on HTT aggregates using striatal-type MSN derived from Huntington's disease-specific iPS cells.
[Figure 18] Figure 18 shows effects of various concentrations of ropinirole which are more effective than bromocriptine in the same concentration range against LDH leakage using striatal-type MSN derived from Huntington's disease-specific iPS cells.
[Figure 19] Figure 19 shows effects of various concentrations of ropinirole on LDH leakage caused by the addition of glutamic acid (50 µM) using striatal-type MSN derived from Huntington's disease-specific iPS cells.
[Figure 20] Figure 20 shows improvement rate of LDH leakage after addition of 300 nM ropinirole to striatal-type MSNs derived from multiple Huntington's disease-specific iPS cells.

### Description of Embodiments

### [Screening of Therapeutic Agent for Huntington's Disease and Pharmaceutical Compositions for Treating Huntington's Disease]

In the present invention, iPS cells are generated from cells collected from patients with Huntington's disease, and striatal medium spiny neurons (MSNs) are differentiated from these iPS cells. Striatal medium spiny neurons (MSNs) that reflect the pathology of Huntington's disease are then used to screen compounds included in the compound library, with the evaluation criteria for their abilities to improve neurite regression, their abilities to suppress HTT aggregate number, and their neuroprotective effect (improvement of neuronal survival rate). This screening allows for the identification of compounds that ameliorate the pathology of striatal medium spiny neurons (MSNs) in Huntington's disease and have a therapeutic effect against Huntington's disease.

A method for inducing differentiation of striatal medium spiny neurons (MSNs) involves inducing differentiation of iPS cells into the lateral basal ganglia primordium by adding Dual Smad inhibition (e.g., LDN-193189 and SB431542) and Wnt inhibition (e.g., Noggin and XAV939) together with Sonic Hedgehog, followed by the addition of Brain-derived neurotrophic factor (BDNF), Glial Cell Line-derived Neurotrophic Factor (GDNF), and ascorbic acid (see, Non-Patent Literature 1). However, because differentiation induction requires a long period of time and its low differentiation efficiency causes intercellular differences, a differentiation induction method using gene expression induction using fibroblasts has been reported (see, Non-Patent Literature 2). However, since this method is based on fibroblasts, the inventor of the present invention has shown that striatal medium spiny neurons (MSNs) can be differentiated in multiple cell lines in a short period of time by inducing gene expression of various transcription factors using iPS cells.

The compound library used for screening can be of any type. For example, a library of compounds that have been clinically tested for various diseases and whose safety has been confirmed can be used.

In the present invention, as a result of screening a compound library using this method, ropinirole, trazodone, rizatriptan, and pyridostigmine were selected as compounds that exhibit neurite regression improvement, HTT aggregate number suppression, and neuroprotective effects (improvement of neuron survival rate). Based on this finding, the inventors of the present invention have completed the present invention.

### [Therapeutic Agent for Huntington's disease, Pharmaceutical composition for treating Huntington's disease]

In the present invention, as pharmaceutical use of a compound represented by the following formula (1-1): 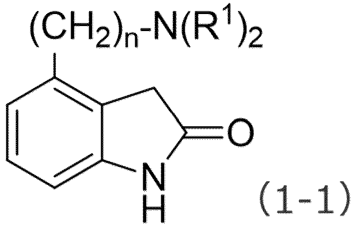
[wherein, in the formula (1-1), R¹ independently represents an alkyl group having 1 to 6 carbon atoms or a 4-hydroxyphenethyl group, and n represents an integer from 1 to 3],
   a compound represented by the following formula (2-1): 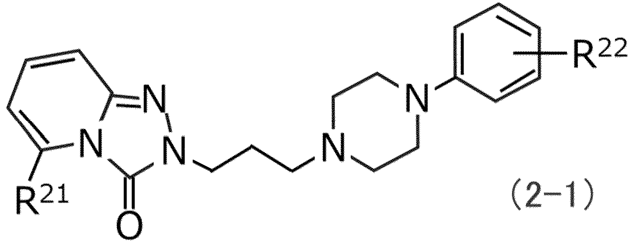
[wherein, in the formula (2-1), R²¹ is H or -CH₃,
R²² is selected from the group consisting of H, an alkyl group having 1 to 6 carbon atoms, an alkoxy group having 1 to 6 carbon atoms, and a halogen],
   a compound represented by the following formula (3-1): 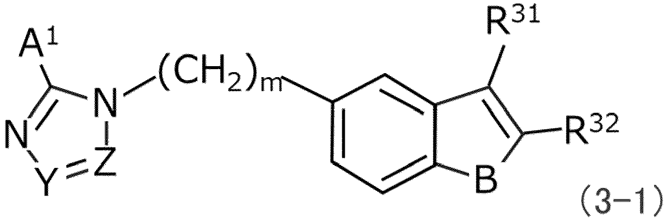
[wherein, in the formula (3-1),
Y represents N or A²-C,
Z represents N or CH,
m is 0, 1, 2 or 3,
B represents O, S or N-R³³,
A¹ and A² independently represent an optionally substituted alkyl group having 1 to 6 carbon atoms, an optionally substituted alkenyl group having 2 to 6 carbon atoms, an optionally substituted alkynyl group having 2 to 6 carbon atoms, an optionally substituted cycloalkyl group having 3 to 7 carbon atoms, an optionally substituted aryl group, an optionally substituted aryl-alkyl group having 1 to 6 carbon atoms, an optionally substituted heterocycloalkyl group having 3 to 7 carbon atoms, an optionally substituted heteroaryl group, an optionally substituted heteroaryl-alkyl group having 1 to 6 carbon atoms, or H, halogen, cyano, trifluoromethyl, an alkoxy group having 1 to 6 carbon atoms, an alkylthio group having 1 to 6 carbon atoms, or -NR^{x}R^{y},
wherein R^{x} and R^{y} independently represent H, a hydrocarbon group, or a heterocyclic group, or R^{x} and R^{y} together represent an alkylene group having 2 to 6 carbon atoms,
R31 represents -CH₂CHR34NR35R36 or the following formula: 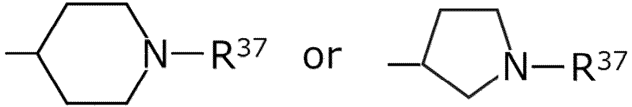
wherein R³², R³³, R³⁴, R³⁵, R³⁶, and R³⁷ independently represent H or an alkyl group having 1 to 6 carbon atoms],
   a compounds represented by the following formula (4-1): 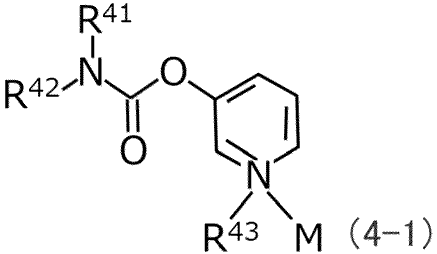
[wherein, in the formula (4-1),
R⁴¹ and R⁴² are selected from an alkyl group having 1 to 6 carbon atoms, an aryl group having 6 to 10 carbon atoms, and an alkyl group having 1 to 6 carbon atoms substituted with an aryl group having 6 to 10 carbon atoms, R⁴³ is selected from an alkyl group having 1 to 6 carbon atoms,
M is selected from halogens and alkyl sulfates]
   or a pharmaceutically acceptable salt thereof, or a solvate thereof, the present invention provides a therapeutic agent for Huntington's disease comprising the above compound, a pharmaceutically acceptable salt thereof, or a solvate thereof, or a pharmaceutical composition for treating Huntington's disease comprising the above compound, a pharmaceutically acceptable salt thereof, or a solvate thereof.

The therapeutic agent for Huntington's disease or the pharmaceutical composition for treating Huntington's disease of the present invention can treat any of the following: motor function symptoms such as chorea and involuntary movements, cognitive function symptoms such as dementia, or psychiatric symptoms. Furthermore, based on the pathological characteristics of patients suffering from Huntington's disease, the therapeutic agent of the present invention can target patients with HTT protein aggregate accumulation, or any neurodegenerative disease accompanied by the accumulation of any protein aggregates of other protein aggregate accumulation abnormalities (e.g., amyloid-beta, alpha-synicrein, TAR DNA-binding protein of 43 kDa, Fused in sarcoma, etc.). In addition, based on the genetic characteristics of patients suffering from Huntington's disease, the therapeutic agent of the present invention can target patients with mutations in the HTT gene, or any other type of Huntington's disease, including patients with mutations in genes that have been reported to accumulate other aggregate proteins (e.g., amyloid-beta, alpha-synicrein, TAR DNA-binding protein of 43 kDa, Fused in sarcoma, etc.).

In the compound of the formula (1-1) above, which is the active ingredient of the therapeutic agent for Huntington's disease or the pharmaceutical composition for treating Huntington's disease of the present invention, in the formula (1-1), n can be 1, 2, or 3. When the active ingredient of the therapeutic agent for Huntington's disease or the pharmaceutical composition for treating Huntington's disease of the present invention is ropinirole, as described below, n in the formula (1-1) is 2. Therefore, the active ingredient of the therapeutic agent for Huntington's disease or the pharmaceutical composition for treating Huntington's disease of the present invention may be a compound in which n in formula (1-1) above is 2.

Furthermore, in the compound of formula (1-1) above, which is the active ingredient of the therapeutic agent for Huntington's disease or the pharmaceutical composition for treating Huntington's disease of the present invention, R¹ in the formula (1-1) above may be a linear, branched, or cyclic alkyl group having 1 to 6 carbon atoms, and more specifically, may be a methyl group, an ethyl group, an n-propyl group, an isopropyl group, an n-butyl group, an isobutyl group, a sec-butyl group, a tert-butyl group, a cyclobutyl group, an n-pentyl group, a cyclopentyl group, an n-hexyl group, and a cyclohexyl group. When the active ingredient of the therapeutic agent for Huntington's disease or the pharmaceutical composition for treating Huntington's disease of the present invention is ropinirole, as described below, R¹ in the formula (1-1) is an n-propyl group. Therefore, the active ingredient of the therapeutic agent for Huntington's disease or the pharmaceutical composition for treating Huntington's disease of the present invention may be a compound in which R¹ in the formula (1-1) above is an n-propyl group.

The compound represented by the formula (1-1) above, which is an active ingredient of the therapeutic agent for Huntington's disease or the pharmaceutical composition for treating Huntington's disease of the present invention, may be 4-[2-(dipropylamino)ethyl]-1,3-dihydro-2H-indol-2-one. That is, the compound represented by the formula (1-1) above may be ropinirole. The chemical formula of ropinirole is shown in the formula (1-2) below.

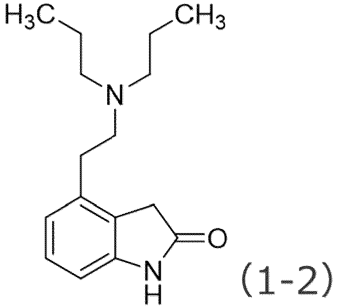

Ropinirole was originally developed as a therapeutic agent for Parkinson's disease due to its dopamine D2 receptor (D2R) agonist activity in dopaminergic neurons. In the present invention, compared to the mechanism of action of ropinirole against Parkinson's disease, it is currently unclear whether ropinirole acts on Huntington's disease based on the same intracellular mechanism of action or a different intracellular mechanism. However, clinical trials regarding ropinirole have already been completed as a pharmaceutical composition, and its safety when administered to living organisms has been fully confirmed. As such, because ropinirole is an existing drug, it is possible to rapidly develop a therapeutic agent for Huntington's disease or a pharmaceutical composition for treating Huntington's disease.

The compound represented by the formula (2-1) above, which is the active ingredient of the therapeutic agent for Huntington's disease or the pharmaceutical composition for treating Huntington's disease of the present invention, may be 2-[3-[4-(m-chlorophenyl)-1-piperazinyl]propyl]-s-triazolo[4,3-a]pyridine-3(2H)-one or its hydrochloride salt. That is, the compound represented by the formula (2-1) above may be trazodone or its hydrochloride salt. The chemical formula for trazodone is shown in formula (2-2) below.

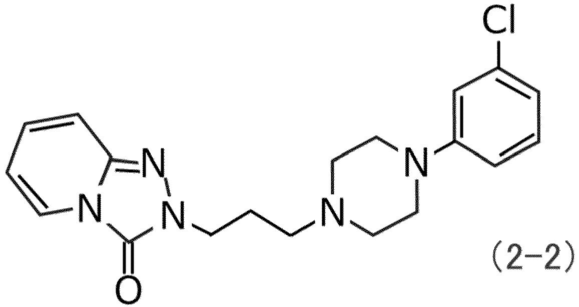

Trazodone or its hydrochloride salt was originally developed as a therapeutic drug for treating depression and depressive states because it exhibits serotonin (5-HT) reuptake inhibitory activity and has the activity to enhance serotonin nerve function, which is reduced in patients with depression. In the present invention, it is currently unclear whether trazodone acts on Huntington's disease based on the same intracellular mechanism of action or a different intracellular mechanism of action. However, clinical trials regarding Trazodone have already been completed as a pharmaceutical composition, and its safety when administered to living organisms has been fully confirmed. As such, since trazodone or its hydrochloride salt is an existing drug, it is possible to rapidly develop a therapeutic agent for Huntington's disease or a pharmaceutical composition for treating Huntington's disease.

The compound represented by the formula (3-1), which is the active ingredient of the therapeutic agent for Huntington's disease or the pharmaceutical composition for treating Huntington's disease in the present invention, may be N,N-dimethyl-2-[5-(1H-1,2,4-triazole-1-ylmethyl)-1H-indole-3-yl]ethaneamine or its benzoate salt. In other words, the compound represented by the formula (3-1) may be lyzatriptan or its benzoate. The chemical formula for lyzatriptan is shown in the formula (3-2) below.

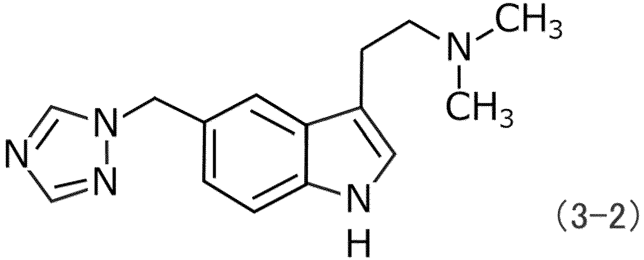

Rizatriptan or its benzoate was originally developed as a therapeutic drug for treating migraines because it acts on 5-HT1B receptors present in cranial blood vessels, selectively constricting extracranial arteries which is thought to dilate during migraine attacks, and also acts on peripheral and central depressant 5-HT1D receptors located o the trigeminal nerve, inhibiting the release of various peptides (such as substance P, calcitonin gene-related peptide), thereby suppressing vasodilation, dural inflammation, and central pain transmission. In the present invention, it is currently unclear whether rizatriptan or its benzoate acts on Huntington's disease based on the same intracellular mechanism of action or a different intracellular mechanism of action. However, clinical trials regarding rizatriptan have already been completed as a pharmaceutical composition, and its safety when administered to living organisms has been fully confirmed. As such, since rizatriptan or its benzoate is an existing drug, it is possible to rapidly develop a therapeutic agent for Huntington's disease or a pharmaceutical composition for treating Huntington's disease.

The compound represented by the formula (4-1), which is the active ingredient of therapeutic agent for Huntington's disease or the pharmaceutical composition for treating Huntington's disease of the present invention, may be a bromide of 3-dimethylcarbamoyloxy-1-methylpyridinium. In other words, the compound represented by the formula (4-1) may be pyridostigmine bromide. The chemical formula of pyridostigmine is shown in the formula (4-2) below.

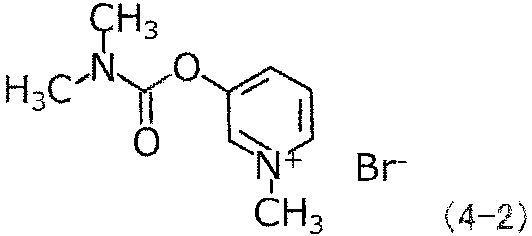

Pyridostigmine bromide was originally developed as a therapeutic drug for treating myasthenia gravis because it reversibly inhibits cholinesterase activity, primarily at the neuromuscular junction, thereby suppressing the breakdown of acetylcholine. As a result, it indirectly enhances the effects of acetylcholine and also possesses acetylcholine-like activity itself. In the present invention, it is currently unclear whether pyridostigmine bromide acts on Huntington's disease based on the same intracellular mechanism of action or a different intracellular mechanism of action. However, clinical trials regarding pyridostigmine bromide have already been completed as a pharmaceutical composition, and its safety when administered to living organisms has been fully confirmed. As such, since pyridostigmine bromide is an existing drug, it is possible to rapidly develop a therapeutic age for Huntington's disease or a pharmaceutical composition for treating Huntington's disease.

The active ingredient of the therapeutic agent for Huntington's disease or the pharmaceutical composition for treating Huntington's disease of the present invention may be a salt of any of the compounds represented by the above formula (1-1), (2-1), (3-1) or (4-1); a solvate of any of the compounds represented by the above formula (1-1), (2-1), (3-1) or (4-1); or a solvate of a salt of any of the compounds represented by the above formula (1-1), (2-1), (3-1) or (4-1).

When a salt of any of the compounds represented by the above formula (1-1), (2-1), (3-1) or (4-1) is used as the active ingredient of the therapeutic agent for Huntington's disease or the pharmaceutical composition for treating Huntington's disease of the present invention, the salt is not particularly limited as long as it is a pharmaceutically acceptable salt, and examples include inorganic acid salts such as hydrochloride, sulfate, hydrobromide, nitrate, and phosphate; organic acid salts such as acetate, mesylate, succinate, maleate, fumarate, citrate, and tartrate; alkali metal salts such as sodium salt and potassium salt; alkaline earth metal salts such as magnesium salt and calcium salt; metal salts such as aluminum salt and zinc salt; ammonium salts such as ammonium salt and tetramethylammonium salt; organic amine addition salts such as morpholine and piperidine; and amino acid addition salts such as glycine, phenylalanine, lysine, aspartic acid, and glutamic acid.

Furthermore, when a solvate of any of the compounds represented by the above formula (1-1), (2-1), (3-1) or (4-1) above or a salt thereof is used in the therapeutic agent for Huntington's disease or the pharmaceutical composition for treating Huntington's disease of the present invention, the solvate is not particularly limited as long as it is a pharmaceutically acceptable solvate, and examples include hydrates, organic solvates, etc.

The active ingredient of the therapeutic agent for Huntington's disease or the pharmaceutical composition for treating Huntington's disease of the present invention may be 4-[2-(dipropylamino)ethyl]-1,3-dihydro-2H-indol-2-one hydrochloride, i.e., ropinirole hydrochloride.

The active ingredient of the therapeutic agent for Huntington's disease or the pharmaceutical composition for treating Huntington's disease of the present invention may be 2-[3-[4-(m-chlorophenyl)-1-piperazinyl]propyl]-s-triazolo[4,3-a]pyridine-3(2H)-one hydrochloride, i.e., trazodone hydrochloride.

The active ingredient of the therapeutic agent for Huntington's disease or the pharmaceutical composition for treating Huntington's disease of the present invention may be N,N-dimethyl-2-[5-(1H-1,2,4-triazole-1-ylmethyl)-1H-indole-3-yl]ethaneamine benzoate, i.e., lyzatriptan benzoate.

The active ingredient of the therapeutic agent for Huntington's disease or the pharmaceutical composition for treating Huntington's disease of the present invention may be 3-dimethylcarbamoyloxy-1-methylpyridinium bromide, i.e., pyridostigmine bromide.

The pharmaceutical composition for treating Huntington's disease of the present invention may be formulated as a pharmaceutical composition and can be administered orally in the form of, for example, tablets, capsules, elixirs, microcapsules, etc., or parenterally in the form of injections, suppositories, topical skin preparations, etc. More specifically, topical skin preparations include dosage forms such as ointments and patches.

In the pharmaceutical composition for treating Huntington's disease of the present invention, any pharmaceutically acceptable carriers typically used in the formulation of pharmaceutical compositions can be used, without particular limitation. More specifically, examples of carriers may include binders such as hypromellose, dextrin, macrogol 400, gelatin, corn starch, tragacanth gum, and gum arabic; excipients such as lactose hydrate, D-mannitol, starch, crystalline cellulose, and alginic acid; solvents for injections such as water, ethanol, and glycerin; and adhesives such as rubber-based adhesives and silicone-based adhesives.

The pharmaceutical composition for treating Huntington's disease of the present invention may contain additives. Examples of additives may include lubricants such as calcium stearate and magnesium stearate; sweeteners such as sucrose, lactose, saccharin, and maltitol; flavoring agents such as peppermint and akamono oil; stabilizers such as carmellose sodium, hydrogenated oil, light anhydrous silicic acid, povidone, glycerin fatty acid ester, benzyl alcohol, and phenol; buffering agents such as phosphate and sodium acetate; solubilizers such as benzyl benzoate and benzyl alcohol; and coloring agents such as yellow ferric oxide, red ferric oxide, black ferric oxide, and titanium oxide.

The pharmaceutical composition for treating Huntington's disease of the present invention can be formulated by appropriately combining the above-mentioned active ingredients with the above-mentioned pharmaceutically acceptable carriers and additives, and mixing them in a unit dosage form required for generally accepted pharmaceutical practice. The pharmaceutical composition for treating Huntington's disease of the present invention may comprise one active ingredient, or two or more active ingredients in combination.

In general, an appropriate daily dose of a pharmaceutical composition for treating Huntington's disease of the present invention is an amount containing the effective minimum dose of the active ingredient to exhibit a therapeutic effect. This effective minimum dose is determined based on various factors, including the activity of the active ingredient contained in the pharmaceutical composition for treating Huntington's disease, functional group modification(s) that determine lipid solubility or water solubility, the route of administration, the duration of administration, the excretion rate of the specific active ingredient used, the duration of treatment, other drugs, compounds, and/or substances used in combination therewith, age, sex, body weight, disease, medical condition, and medical history of the patient, and other factors well known in the medical arts. Typically, the dose of the pharmaceutical composition for treating Huntington's disease of the present invention administered to a patient is an amount including the effective ingredient at about 0.0001 to about 100 mg/kg of body weight per day. The pharmaceutical composition for treating Huntington's disease of the present invention may be administered once a day or in divided doses about 2 to 4 times a day.

In particular, in a case where the active ingredient is a compound represented by the formula (1-2), the dosage of the pharmaceutical composition for treating Huntington's disease of the present invention is considered to be orally administered at a dose of 2 mg of the active ingredient once day, for example, as a sustained-release ropinirole formulation, and is administered orally, with the dose increased weekly to no more than 16-24 mg of active ingredient per day.

In particular, in a case where the active ingredient is a compound represented by the formula (2-2), the dosage of the pharmaceutical composition for treating Huntington's disease of the present invention is considered to be orally administered, for example, at a dose of 25 mg of the active ingredient in 3 divided doses per day, and is gradually increasing the dose so as not to exceed a daily dose of 75-200 mg of the active ingredient.

In particular, in a case where the active ingredient is a compound represented by the formula (3-2), the dosage of the pharmaceutical composition for treating Huntington's disease of the present invention is considered to be orally administered, for example, at a dose of 10 mg of the active ingredient once a day, with the dosage increasing every week, and the daily dose should not exceed 10 to 20 mg of the active ingredient.

In particular, when the active ingredient is a compound represented by the formula (4-2), the dosage of the pharmaceutical composition for treating Huntington's disease of the present invention is considered to be orally administered, for example, at a dose of 60 mg of the active ingredient once a day, and should not exceed a daily dose of 180 mg of the active ingredient.

### [Other Embodiments]

In another embodiment, the present invention provides a method for treating Huntington's disease, comprising administering to a patient in need of such treatment any one of the compounds represented by the formula (1-1), (2-1), (3-1), or (4-1), or a pharmaceutically acceptable salt thereof, or a solvate thereof. In this embodiment of the present invention, the active ingredient may be any one of the compounds represented by the formula (1-1), (2-1), (3-1), or (4-1), or a pharmaceutically acceptable salt thereof, or a solvate thereof, as described above. Furthermore, in this embodiment of the present invention, the dosage of the active ingredient can be determined by considering the appropriate dosage for each compound. For example, the dose of ropinirole used as the active ingredient is as described above.

The present invention provides a compound represented by the above formula (1-1), a compound represented by the above formula (2-1), a compound represented by the above formula (3-1), or a compound represented by the above formula (4-1), or a pharmaceutically acceptable salt thereof, or a solvate thereof, for treating Huntington's disease. In this embodiment of the present invention, the compound represented by the above formula (1-1), a compound represented by the above formula (2-1), a compound represented by the above formula (3-1), or a compound represented by the above formula (4-1), or a pharmaceutically acceptable salt thereof, or a solvate thereof, can be the same as those described above.

The following examples illustrate the present invention in more detail. The following examples are not intended to limit the present invention in any way.

### Examples

### Example 1: Screening of compounds for treating Huntington's disease

In this example, disease-specific iPS cells derived from cells of Huntington's disease patients were used to induce differentiation into striatal-type medium spiny neurons (MSNs) that can be used for screening compounds for treating Huntington's disease, and compounds that can improve the pathological condition of Huntington's disease in these striatal-type MSNs were screened.

### (1-1) Differentiation and induction of striatal-type medium spiny neurons (MSNs)

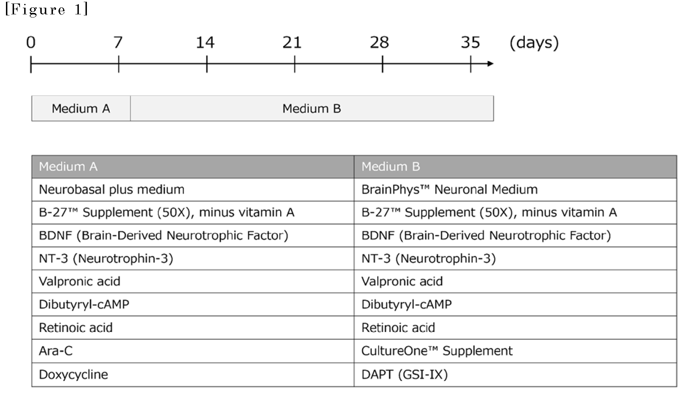

Huntington's disease is known to have various pathological conditions due to an increase in the number of CAG repeats in the huntingtin gene (HTT gene). In the present invention, iPS cells derived from individual patients with various CAG repeat counts (5 lines) as disease-specific iPS cells, and iPS cells derived from healthy individuals (3 lines) were used to induce differentiation into striatal-type MSNs based on the culture method shown in Figure 1.

The iPS cells used in this specification are as follows: Disease-specific iPS cells
HD1.CAG180; 180 CAG repeats
HD2.CAG50; 50 CAG repeats
HD3.CAG47; 47 CAG repeats
HD4.CAG50; 50 CAG repeats
HD5.CAG42; 42 CAG repeats
iPS cells derived from healthy individuals
ND50018:
   ND50025:
   RC802:

Specifically, first, each cell line, which had plasmids containing the microRNA9/124, ASCL1, BCL2L1, CTIP2, MYT1L, and FOXP1/2 genes linked to a promoter whose expression is induced in the presence of Doxycycline (DOX), incorporated using the PiggyBac method, was induced for 7 days with a final concentration of 2 µg/mL of Doxycycline (CAS number: 24390-14-5) to express the transcription factors ASCL1, BCL2L1, CTIP2, MYT1L, and FOXP1/2 proteins, which are expressed in the early stages of neuronal differentiation, while simultaneously culturing for 7 days in Neurobasal plus (Trademark) medium (Thermo Fischer Scientific A3582901) (Medium A) containing a final concentration of 10 ng/ml of BDNF (CAS No.: 218441-99-7), a final concentration of 10 ng/ml of neurotrophin-3 (NT-3) (CAS No.: 130939-66-1), a final concentration of 1 microM of valproic acid (CAS No.: 1069-66-5), a final concentration of 200 microM of dibutyl-cAMP (dbcAMP) (CAS No.: 16980-89-5), a final concentration of 1 microM of retinoic acid (RA) (CAS No.: 302-79-4), and a final concentration of 2% of B27 (Trademark) Supplement (Thermo Fischer Scientific A3582901) (Medium A), to induce striatal-type medium spiny neurons (MSNs). During the culture period, on days 1 and 3, 2 microM cytarabine (Ara-C) was added to the culture medium to stop astrocyte growth. The culture medium was changed every 2-3 days.

Next, the cells were cultured for 28 days until Day 35 Brainphys (Trademark) Neuronal Medium (STEMCELL Technologies, ST-05790) (Medium B) containing a final concentration of 10 ng/ml of BDNF (CAS No.: 218441-99-7), a final concentration of 10 ng/ml Neurotrophin-3 (NT-3) (CAS No.: 130939-66-1), a final concentration of 1 microM of valproic acid (CAS No.: 1069-66-5), a final concentration of 200 microM of Dibutyl-cAMP (dbcAMP) (CAS No.: 16980-89-5), a final concentration of 2% of retinoic acid (RA) (CAS No.: 302-79-4), a final concentration of 2% of B27 (Trademark) Supplement (Thermo Fischer Scientific, 12587010), and a final concentration of 0.1% of CultureOne (Trademark) Supplement (Gibco, A3320201). Only when changing the medium on Day 8, a final concentration of 1 microM of DAPT (CAS number: 208255-80-5) was added to the medium. The medium was changed every 2-3 days.

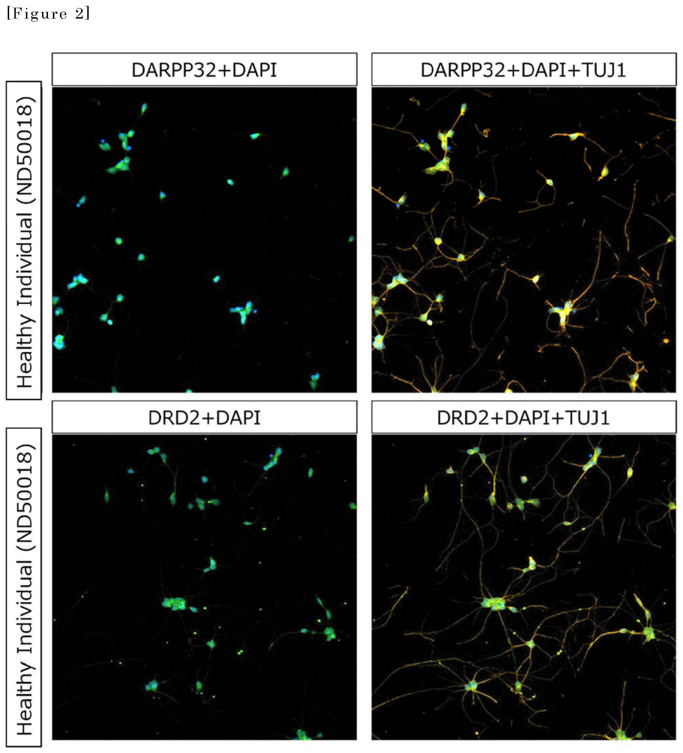

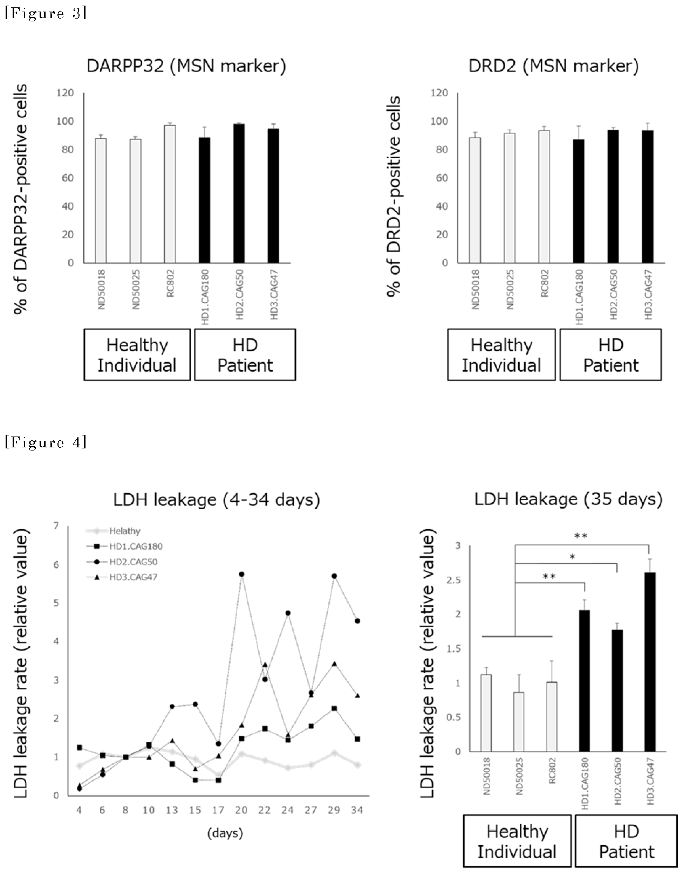

The differentiated cells derived from healthy individuals were immunostained, and the expression of MSN markers DARPP32 and DRD2 were examined. The results are shown in Figure 2. The results showed that DARPP32 and DRD2 were expressed together with TUJ1, a panneuronal cell marker. When the percentage of DARPP32 and DRD2-positive cells was examined from the obtained neurons, the percentage was similar in MSNs differentiated from iPS cells derived from Huntington's disease patients as in MSNs differentiated from iPS cells derived from healthy individuals, which are shown in Figure 3. These results indicate that the cells differentiated using the above procedure are striatal type MSNs.

Furthermore, the present inventor confirmed whether differentiated striatal-type MSNs derived from Huntington's disease-specific iPS cells exhibited physiological indicators specific to Huntington's disease. As physiological indicators specific to Huntington's disease, the present inventor measured lactate dehydrogenase (LDH) leakage rate, neurite length reduction, and HTT aggregate accumulation, which are markers of neuronal cell death. The LDH leakage rate was measured using a commercially available kit (model "G7891", Promega). The neurite length was quantitatively measured up to 35 days over time after the start of culture, by immunostaining using TUJ1 antibody on Days 7, 14, 21, 28, and 35, and analyzing image of the immunostain as an indicator. The accumulation of HTT aggregates was quantitatively measured up to 35 days over time after the start of culture, by immunostaining using HTT antibody and polyglutamine antibody on Days 7, 14, 21, 28, and 35. and analyzing Image of the immunostain as an indicator. All measured values were quantified as a ratio to the average value obtained from MSNs created from iPS cells derived from healthy individuals.

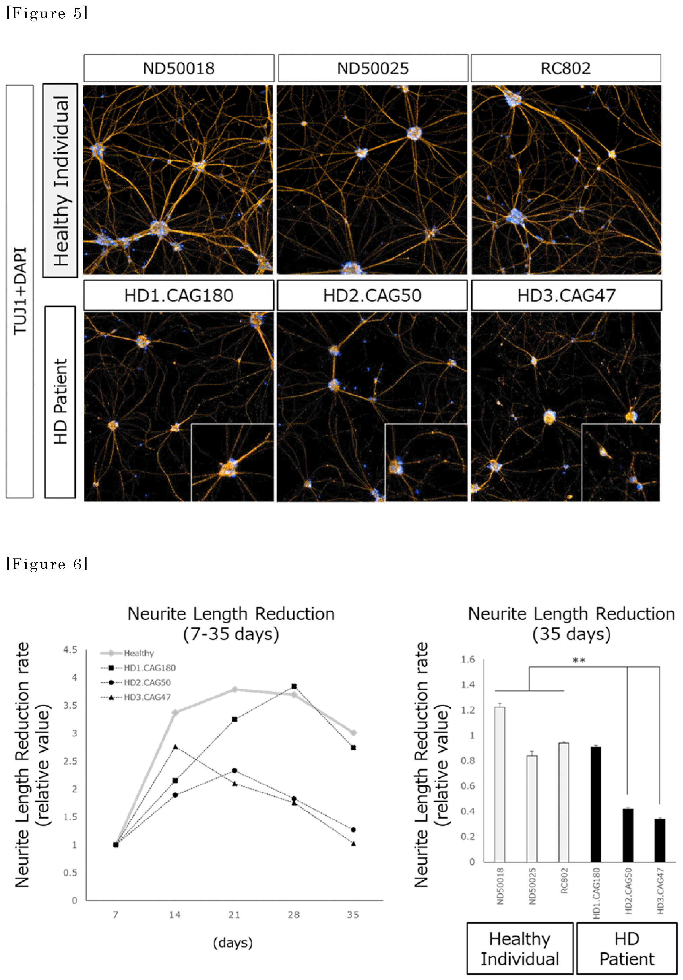

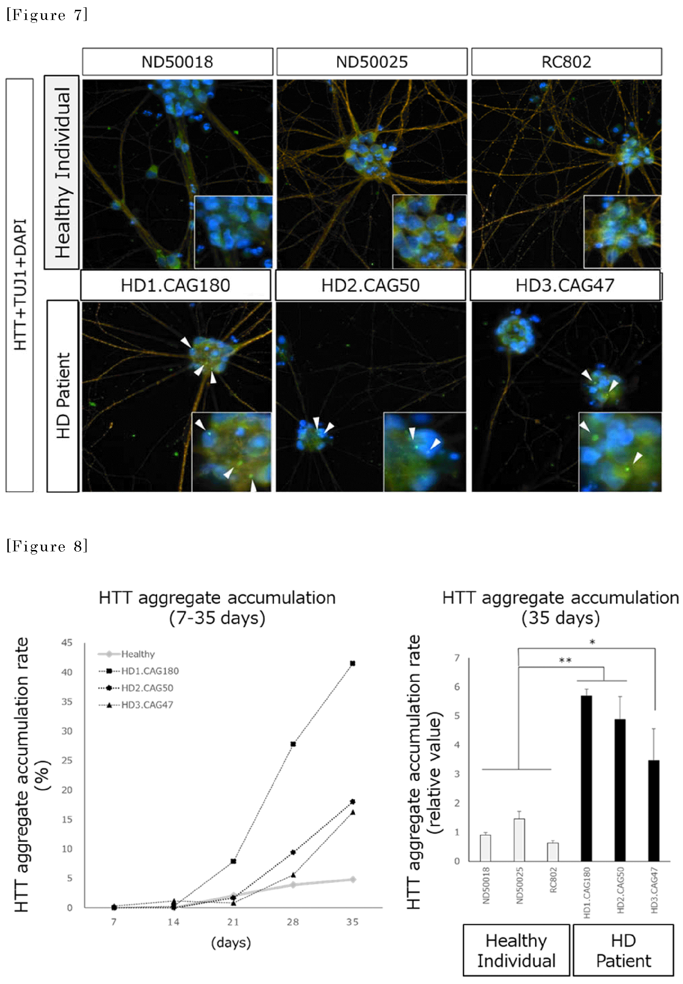

As a result, compared to the results obtained from MSNs differentiated from iPS cells derived from healthy individuals, all striatal-type MSNs derived from Huntington's disease-specific iPS cells showed a significantly increased LDH leakage rate (Figure 4), a significant reduction of neurite length (Figures 5 and 6), and a significant accumulation of HTT aggregates (Figures 7 and 8) (Figures 5 and 7 are both obtained using cells of day 35). These results indicate that the differentiated MSN cells exhibit the pathology characteristic of Huntington's disease as a phenotype. In Figures 4, 6, and 8, "*" and "**" indicate that a statistically significant difference exists at a significance level of less than 5% or less than 1%, respectively.

### (1-2) Results of drug screening using striatal-type medium spiny neurons (MSNs)

Using striatal-type MSNs derived from Huntington's disease-specific iPS cells differentiated in (1-1) above, drugs that restore the Huntington's disease phenotype were screened from an existing drug library, using the reduction in LDH leakage rate, the neurite length elongation, and the reduction in HTT aggregate accumulation, as indicators, the phenotypes as shown in (1-1). The LDH leakage rate measurement, the neurite length measurement, and the HTT aggregate quantification were performed using the same methods as described in Example (1-1). For screening, two types of iPS cells, HD1.CAG180 and HD2.CAG50, were used.

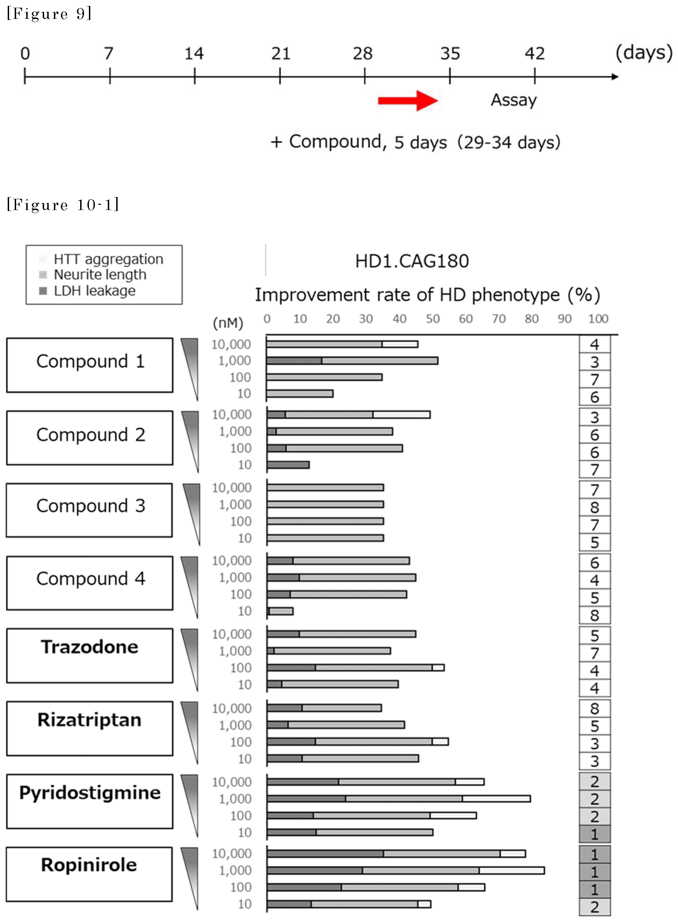

As shown in Figure 9, each compound from the existing drug library was added to the culture medium 29-34 days after the start of differentiation induction, and then screened. As a result, eight drugs were found as promising therapeutic agents for Huntington's disease. The improvement rate (%) in the Huntington's disease phenotype when these drugs were added to the culture medium was calculated.

The improvement rate (%) of the Huntington's disease phenotype was calculated for each parameter using the following formula (1): $Improvement rate \left(%\right) = \left(A-B\right) / \left(A-C\right) \times 100$ (1) [In the formula (1),
"A" represents the measured value of striatal-type medium spiny neurons (MSNs) differentiated from iPS cells derived from HD patients in the absence of drugs,
"B" represents the measured value of striatal-type medium spiny neurons (MSNs) differentiated from iPS cells derived from HD patients in the presence of drugs,
"C" represents the measured value of striatal-type medium spiny neurons (MSNs) differentiated from iPS cells derived from healthy individuals in the absence of drugs.]

The percentages of each phenotype -LDH leakage, neurite regression, and HTT aggregate accumulation- were set to a maximum of 35%, 35%, and 30%, respectively, so that the total would be 100%.

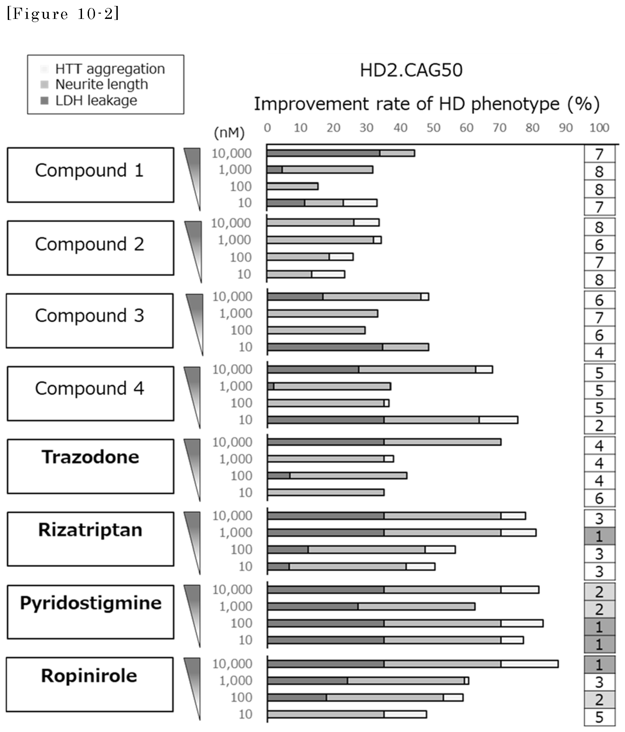

Figure 10 shows the results of adding the eight drugs obtained above at final concentrations of 0.01 microM, 0.1 microM, 1 microM, and 10 microM to the culture medium of striatal-type medium spiny neurons (MSNs) differentiated from iPS cells derived from HD patients (HD1.CAG180 and HD2.CAG50). As a result, ropinirole, trazodone, rizatriptan, and pyridostigmine showed high neuroprotective effects when measured in the range of 0.01 microM to 10 microM, calculated as the improvement rate (%) in Huntington's disease phenotype, with the total percentage of each phenotype (LDH leakage, neurite retraction, and HTT aggregate accumulation) considered to be 100%.

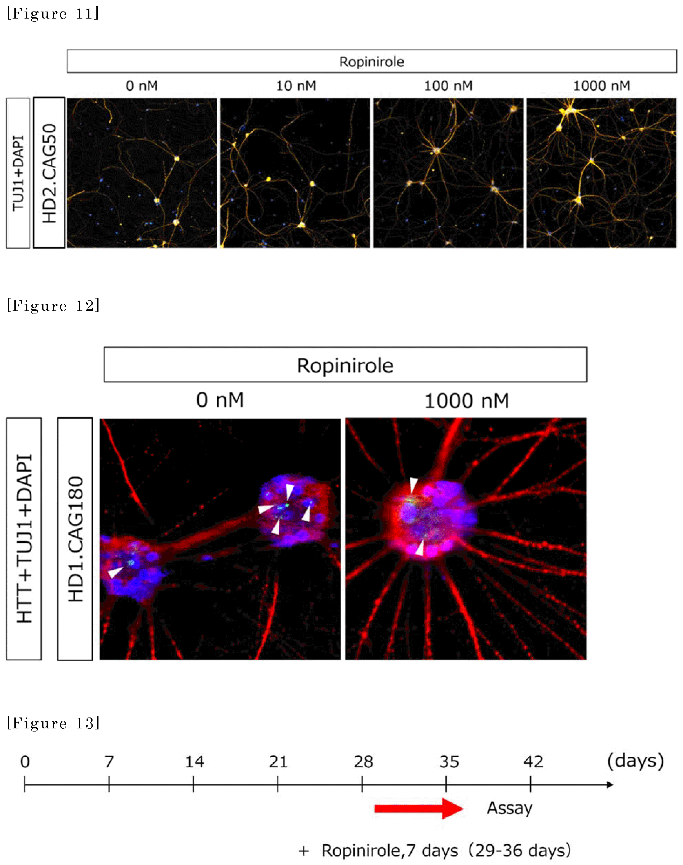

Based on the results, ropinirole was selected for further investigation from among these compounds. Immunostaining of cells also showed that ropinirole exhibits improvement effect on neurite regression (see, Figure 11, using HD2.CAG50 cells) and improvement of HTT aggregates (see, Figure 12, using HD1.CAG180 cells). Table 1 shows the improvement rates of ropinirole on each phenotype for both cell types (HD1.CAG180 and HD2.CAG50).

**[Table 1]**

| | HD1.CAG180 | | | |
|---|---|---|---|---|
| | 0.01 microM | 0.1 microM | 1 microM | 10 microM |
| Improvement rate (%) of LDH leakage | 13.3 | 22.4 | 28.5 | 35.0 |
| Improvement rate (%) of neurite retraction | 31.9 | 35.0 | 35.0 | 35.0 |
| Improvement rate (%) of HTT aggregate accumulation | 3.7 | 7.9 | 19.8 | 7.4 |
| Total (%) | 49.0 | 65.2 | 83.3 | 77.4 |

| | HD2.CAG50 | | | |
|---|---|---|---|---|
| | 0.01 microM | 0.1 microM | 1 microM | 10 microM |
| Improvement rate (%) of LDH leakage | 0.0 | 17.7 | 23.9 | 35.0 |
| Improvement rate (%) of neurite retraction | 35.0 | 35.0 | 35.0 | 35.0 |
| Improvement rate (%) of HTT aggregate accumulation | 12.6 | 5.8 | 1.2 | 17.0 |
| Total (%) | 47.6 | 58.5 | 60.2 | 87.0 |

### Example 2: Function of Ropinirole

In this example, the ropinirole obtained in Example 1 (1-2) was used to analyze function on striatal-type medium spiny neurons (MSNs) differentiated from iPS cells derived from Huntington's disease patients.

### (2-1) Procedure for cell treatment with ropinirole

The therapeutic effects of ropinirole were evaluated in the same manner as in Example 1 (1-2), except that the timing of ropinirole addition in culture process was changed to 29-36 days from the start of differentiation induction, as shown in Figure 13.

Specifically, ropinirole was added on days 29, 31 and 34 to the culture medium of striatal-type medium spiny neurons (MSNs) differentiated from iPS cells derived from Huntington's disease patients having HTT gene mutations (HD1.CAG180, HD2.CAG50, and HD3.CAG47) and the assay was performed on day 36. Ropinirole was added to the culture medium at final concentrations of 10 nM, 100 nM, and 1000 nM.

### (2-2) Neuroprotective effect of ropinirole

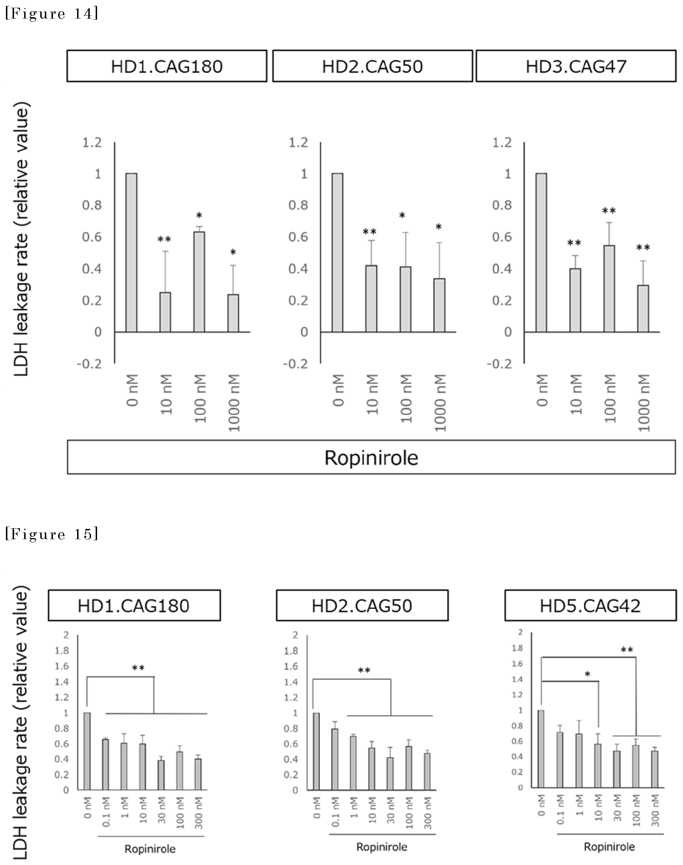

As a parameter of neuroprotective effect, the LDH leakage rate induced was measured after addition of ropinirole. The LDH leakage rate was measured in the same manner as in Example 1 (1-2). The results are shown in Figures 14 and 15.

Figure 14 is a graph showing the LDH leakage rate measured when various concentrations (10 nM, 100 nM, 1000 nM) of ropinirole were added to the culture medium of striatal-type medium spiny neurons (MSNs) differentiated from iPS cells derived from Huntington's disease patients with different CAG repeat numbers (HD1.CAG180, HD2.CAG50, and HD3.CAG47). The vertical axis of Figure 14 shows the LDH leakage rate (relative value) when ropinirole is added at various concentrations, which are compared to the LDH leakage value under conditions without addition of ropinirole as the standard. The horizontal axis of Figure 14 shows the results when ropinirole was added to the culture medium at a concentration of 10 nM, 100 nM, or 1000 nM. In Figure 14, the symbol "*" indicates a statistically significant difference at a significance level of less than 5%, and the symbol "**" indicates a statistically significant difference at a significance level of less than 1%.

Figure 15 is a graph showing the LDH leakage rate measured when various concentrations (0.1 nM, 1 nM, 10 nM, 30 nM, 100 nM, 300 nM) of ropinirole, in more detail than in the experiment in Figure 14, were added to the culture medium of striatal-type medium spiny neurons (MSNs) differentiated from iPS cells derived from Huntington's disease patients with different CAG repeat numbers (HD1.CAG180, HD2.CAG50, and HD5.CAG42). The vertical axis of Figure 15 shows the LDH leakage rate (relative value) when ropinirole was added at various concentrations, which are compared to the LDH leakage value under conditions without addition of ropinirole as the standard, and the horizontal axis of Figure 15 shows the results when ropinirole was added to the culture medium at a concentration of 0.1 nM, 1 nM, 10 nM, 30 nM, 100 nM, or 300 nM. In Figure 15, the symbol "*" indicates a statistically significant difference at a significance level of less than 5%, and the symbol "**" indicates a statistically significant difference at a significance level of less than 1%.

The results of Figures 14 and 15 show that, even at 29-36 days after the start of differentiation induction, the addition of ropinirole exhibited the neuroprotective effects (i.e., reduction of LDH leakage). Furthermore, EC50 of ropinirole in Figure 14 was less than 10 nM (calculated using ImageJ and Excel), and IC50 of ropinirole in Figure 15 was 64.2 nM (calculated using ImageJ and Excel), demonstrating that neuroprotective effects can be exerted at very low doses.

### (2-3) Improvement effect of ropinirole on HTT aggregate accumulation

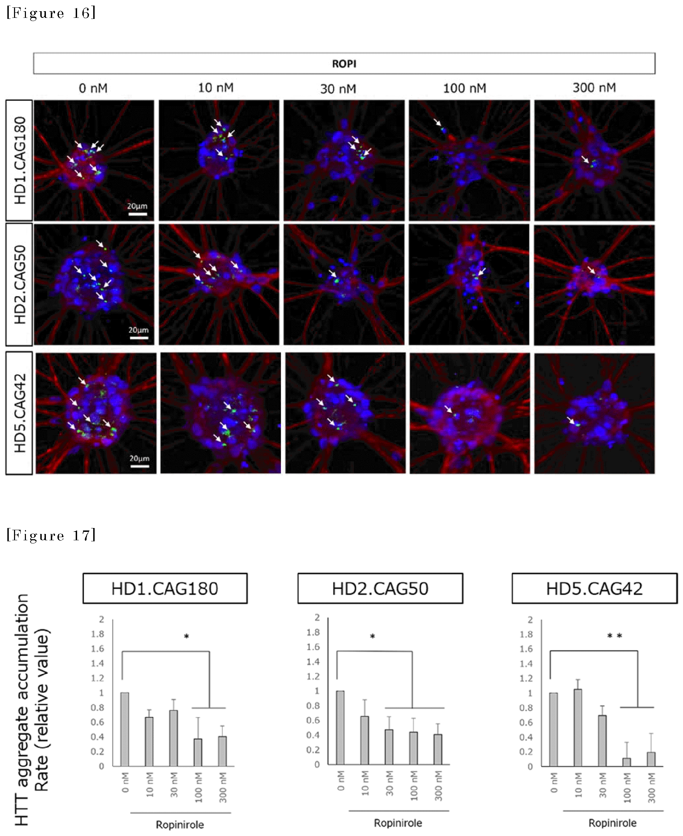

Regarding the accumulation of HTT aggregates, a physiological indicator unique to Huntington's disease, in striatal-type medium spiny neurons (MSNs) differentiated from iPS cells derived from Huntington's disease patients, the change in the HTT aggregate accumulation rate after addition of ropinirole was measured as a parameter for the improvement effect on HTT aggregate accumulation. The measurement of the HTT aggregate accumulation rate was performed in the same manner as in Example 1. The results are shown in Figures 16 and 17.

Figure 16 is a graph showing the improvement of HTT aggregate accumulation by immunostaining when various concentrations (10 nM, 30 nM, 100 nM, 300 nM) of ropinirole were added to the culture medium of striatal-type medium spiny neurons (MSNs) differentiated from iPS cells derived from Huntington's disease patients with different CAG repeat numbers (HD1.CAG180, HD2.CAG50, or HD5.CAG42). The arrows in Figure 16 indicate the HTT aggregate accumulation sites, and the horizontal axis of Figure 16 shows the results of adding ropinirole to the culture medium at concentrations of 10 nM, 30 nM, 100 nM, and 300 nM.

Figure 17 is a graph a graph that quantifies the results from Figure 16. The vertical axis of Figure 17 shows the relative accumulation rate of HTT aggregates when ropinirole is added at various concentrations, which are compared to the number of HTTs under conditions without addition of ropinirole as the standard. The horizontal axis under conditions without addition of ropinirole as the standard. shows the results when ropinirole is added to the culture medium at a concentration of 10 nM, 30 nM, 100 nM, or 300 nM. In Figure 17, the symbol "*" indicates a statistically significant difference at a significance level of less than 5%, and the symbol "**" indicates a statistically significant difference at a significance level of less than 1%.

The results of Figures 16 and 17 show that, even at days 29-36 after the start of differentiation induction, the addition of ropinirole exhibited improvement effect on HTT aggregate accumulation (i.e., reduction of HTT aggregate accumulation rate). Furthermore, IC50 of ropinirole was 40.5 nM (calculated using ImageJ and Excel), demonstrating that it can exert an improvement effect on HTT aggregate accumulation at very low doses.

### (2-4) Neuroprotective effect of ropinirole compared with similar agonists

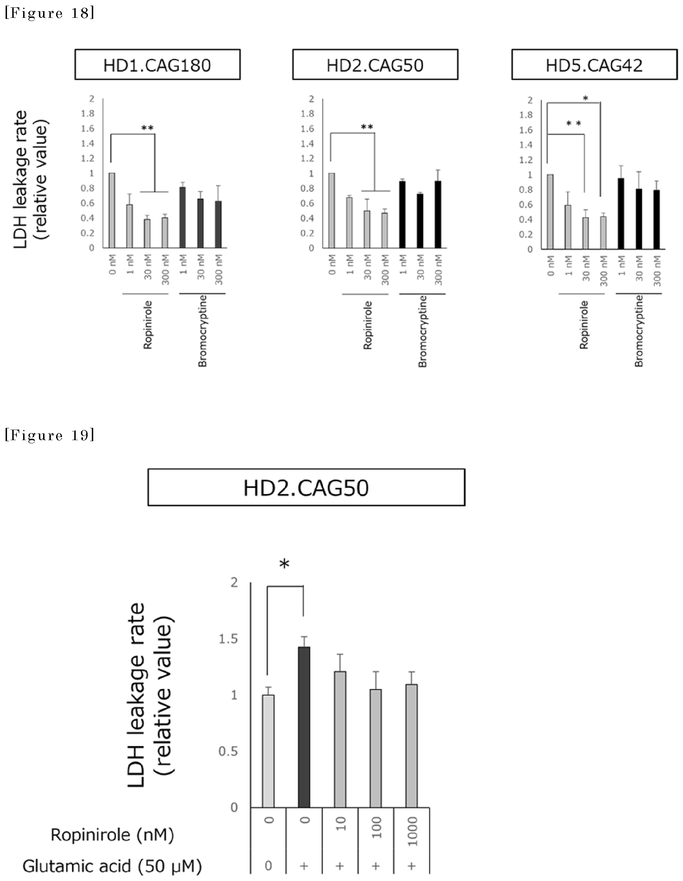

Since ropinirole possesses dopamine D2 receptor (D2R) agonist activity in dopamine neurons, the neuroprotective effect of ropinirole was compared with that of bromocriptine, which is also a D2 receptor agonist. As a parameter of neuroprotective effect, the LDH leakage rate after addition of ropinirole was measured. The LDH leakage rate was measured in the same manner as in Example 1. The results are shown in Figure 18.

Figure 18 is a graph showing the results of the LDH leakage rate measured when various concentrations of ropinirole or various concentrations of bromocriptine (D2 receptor agonist) were added to the culture medium of striatal-type medium spiny neurons (MSNs) differentiated from iPS cells derived from Huntington's disease patients (HD1.CAG180, HD2.CAG50, and HD5.CAG42). The vertical axis of Figure 18 shows the LDH leakage rate (relative value) when ropinirole was added at various concentrations, which are compared to the LDH leakage value under conditions without addition of ropinirole as the standard. The horizontal axis of Figure 18 shows the results when ropinirole was added to the culture medium at a concentration of 1 nM, 30 nM, or 300 nM, or when bromocriptine was added at 1 nM, 30 nM, or 300 nM. In Figure 15, the symbol "*" indicates a statistically significant difference at a significance level of less than 5%, and the symbol "**" indicates a statistically significant difference at a significance level of less than 1%.

As a result, on days 29-36 after the start of differentiation induction, the addition of ropinirole exhibited the neuroprotective effect (i.e., reduction of LDH leakage), but the addition of bromocriptine exhibited no neuroprotective effect. This result indicates that ropinirole, a D2 receptor agonist, exhibits higher neuroprotective effect than bromocriptine, another D2 receptor agonist.

### (2-5) Neuroprotective effect of ropinirole on glutamate-induced cell death

It is known that excessive stimulation of nerve cells with glutamate can cause overexcitement of the nerve cells, leading to voluntary cell death. This phenomenon, known as glutamate-induced neuroexcitotoxicity, is thought to be a cause of various neurodegenerative diseases. To confirm whether ropinirole has a neuroprotective effect on glutamate-induced neuroexcitotoxicity, the LDH leakage rate after addition of ropinirole was measured as a parameter of neuroprotective effect. The LDH leakage rate was measured in the same manner as in Example 1. The results are shown in Figure 19.

Figure 19 is a graph showing the LDH leakage rate measured when glutamate and various concentrations of ropinirole were added to the culture medium of striatal-type medium spiny neurons (MSNs) differentiated from iPS cells derived from Huntington's disease patient (HD2.CAG50). The vertical axis of Figure 19 shows the relative LDH leakage rates when ropinirole is added at various concentrations to cells with 50 microM glutamate, which are compared to the LDH leakage value under conditions without addition of glutamate or ropinirole as the standard. The horizontal axis of Figure 19 shows the results when ropinirole is added to the culture medium at a concentration of 10 nM, 100 nM, or 1000 nM. The symbol "+" on the horizontal axis of Figure 19 indicates that the culture medium was supplemented with 50 µM glutamate. The symbol "*" in Figure 19 indicates a statistically significant difference at a significance level of less than 5%, and the symbol "**" indicates a statistically significant difference at a significance level of less than 1%.

As a result, on days 29-36 after the start of differentiation induction, neuronal cell death was observed when glutamate was added, but the addition of ropinirole exhibited the neuroprotective effects (i.e., reduction in LDH leakage). This result indicates that ropinirole exerts neuroprotective effects against excessive stimulation of glutamate receptors.

### (2-6) Neuroprotective effects of ropinirole using various HD lines

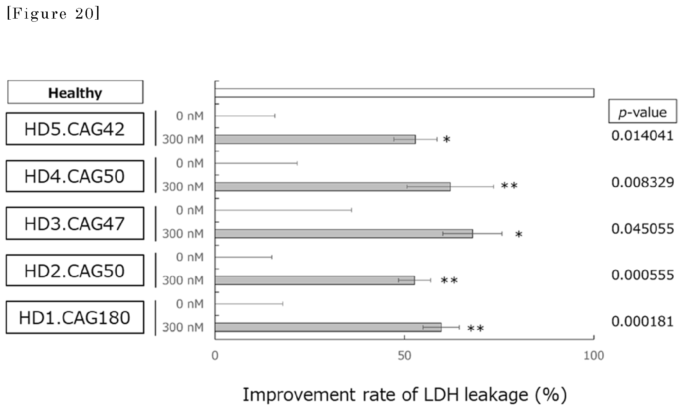

As a parameter of the neuroprotective effect of addition of ropinirole, the LDH leakage rate induced by ropinirole administration was measured, and the improvement effect of addition of ropinirole on the LDH leakage rate was examined based on the change in the LDH leakage rate. The improvement rate of the LDH leakage was calculated in the same manner as in Example 1 (1-2). The results are shown in Figure 20.

Figure 20 is a graph showing the improvement rate of LDH leakage after addition of ropinirole, based on the results of the LDH leakage rate, which were measured when 300 nM of ropinirole was added to the culture medium of striatal-type medium spiny neurons (MSNs) differentiated from iPS cells derived from Huntington's disease patients (HD1.CAG180, HD2.CAG50, HD3.CAG47, HD4.CAG50, and HD5.CAG42). The horizontal axis of Figure 20 shows the improvement rate (relative value) of LDH leakage from the LDH leakage value before addition of ropinirole (0 nM) to the LDH leakage value after addition of ropinirole (300 nM), which are compared to the LDH leakage value in a healthy human sample as the standard (100%), and the vertical axis of Figure 20 shows the results of addition of ropinirole to the culture medium for each cell (0 nM or 300 nM). In Figure 20, the symbol "*" indicates a statistically significant difference at a significance level of less than 5%, and the symbol "**" indicates a statistically significant difference at a significance level of less than 1%.

As a result, on days 29-36 after the start of differentiation induction, the addition of ropinirole exhibited neuroprotective effects (i.e., improvement of LDH leakage). This result indicates that the neuroprotective effect of ropinirole is effective in treating patients with a wide range of HTT CAG repeat counts.

### Industrial Applicability

According to the present invention, a therapeutic agent for Huntington's disease and a therapeutic composition for Huntington's disease can be provided. The therapeutic agent for Huntington's disease or the therapeutic composition for Huntington's disease of the present invention can be used not only to treat Huntington's disease but also to prevent the onset of Huntington's disease. Furthermore, by analyzing the mechanism of action of the therapeutic agent for Huntington's disease of the present invention on striatal-type medium spiny neurons (MSNs) differentiated from iPS cells derived from Huntington's disease patients, the pathogenesis of Huntington's disease can be elucidated.

## Claims

1. A therapeutic agent for Huntington's disease comprising:
a compound represented by the following formula (1-1): 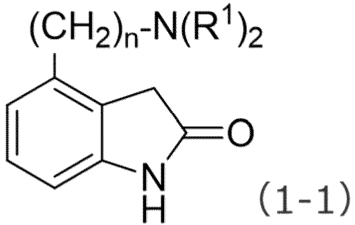 [wherein, in the formula (1-1), R¹ independently represents an alkyl group having 1 to 6 carbon atoms or a 4-hydroxyphenethyl group, and n represents an integer from 1 to 3],
a compound represented by the following formula (2-1): 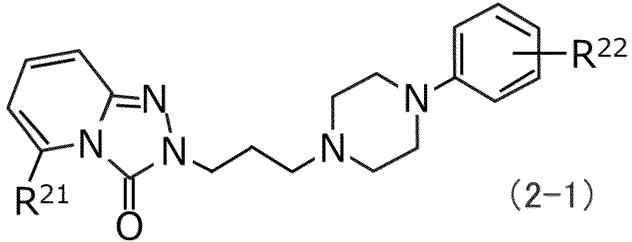
[wherein, in the formula (2-1), R²¹ is H or -CH₃,
R²² is selected from the group consisting of H, an alkyl group having 1 to 6 carbon atoms, an alkoxy group having 1 to 6 carbon atoms, and a halogen],
a compound represented by the following formula (3-1): 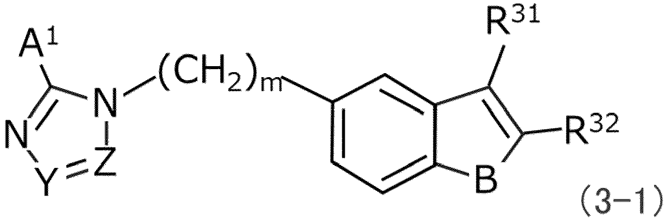
[wherein, in the formula (3-1),
Y represents N or A²-C,
Z represents N or CH,
m is 0, 1, 2 or 3,
B represents O, S or N-R³³,
A¹ and A² independently represent an optionally substituted alkyl group having 1 to 6 carbon atoms, an optionally substituted alkenyl group having 2 to 6 carbon atoms, an optionally substituted alkynyl group having 2 to 6 carbon atoms, an optionally substituted cycloalkyl group having 3 to 7 carbon atoms, an optionally substituted aryl group, an optionally substituted aryl-alkyl group having 1 to 6 carbon atoms, an optionally substituted heterocycloalkyl group having 3 to 7 carbon atoms, an optionally substituted heteroaryl group, an optionally substituted heteroaryl-alkyl group having 1 to 6 carbon atoms, or H, halogen, cyano, trifluoromethyl, an alkoxy group having 1 to 6 carbon atoms, an alkylthio group having 1 to 6 carbon atoms, or -NR^{x}R^{y},
wherein R^{x} and R^{y} independently represent H, a hydrocarbon group, or a heterocyclic group, or R^{x} and R^{y} together represent an alkylene group having 2 to 6 carbon atoms,
R³¹ represents -CH₂CHR³⁴NR³⁵R³⁶ or the following formula: 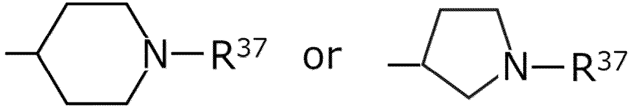
wherein R³², R³³, R³⁴, R³⁵, R³⁶, and R³⁷ independently represent H or an alkyl group having 1 to 6 carbon atoms],
a compounds represented by the following formula (4-1): 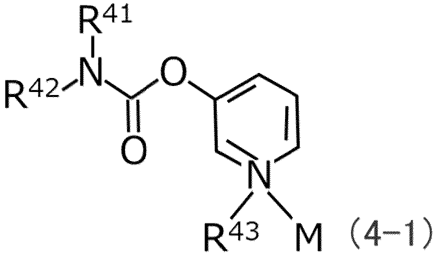
[wherein, in the formula (4-1),
R⁴¹ and R⁴² are selected from an alkyl group having 1 to 6 carbon atoms, an aryl group having 6 to 10 carbon atoms, and an alkyl group having 1 to 6 carbon atoms substituted with an aryl group having 6 to 10 carbon atoms, R⁴³ is selected from an alkyl group having 1 to 6 carbon atoms,
M is selected from halogens and alkyl sulfates]
or a pharmaceutically acceptable salt thereof, or a solvate thereof.

2. The therapeutic agent for Huntington's disease according to Claim 1, wherein n is 2 in the formula (1-1).

3. The therapeutic agent for Huntington's disease according to Claim 1 or 2, wherein R¹ in the formula (1-1) is an n-propyl group.

4. The therapeutic agent for Huntington's disease according to Claim 1 or 2, wherein the compound represented by the formula (1-1) is a compound represented by the following formula (1-2): 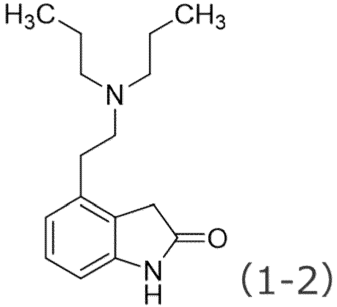 (4-[2-(dipropylamino)ethyl]-1,3-dihydro-2H-indole-2-one).

5. The therapeutic agent for Huntington's disease according to Claim 4, wherein the pharmaceutically acceptable salt of the compound represented by the formula (1-1) is a hydrochloride salt of the compound represented by the formula (1-2) (4-[2-(dipropylamino)ethyl]-1,3-dihydro-2H-indole-2-one hydrochloride).

6. The therapeutic agent for Huntington's disease according to Claim 1 or 2, wherein the compound represented by the formula (2-1) is a compound represented by the following formula (2-2): 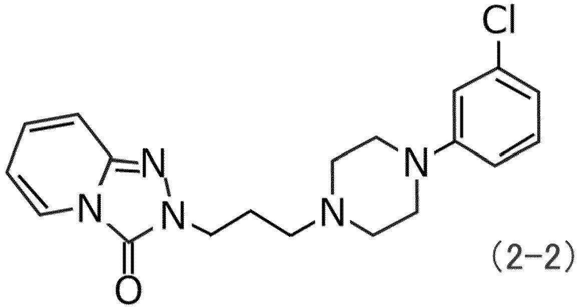 (2-[3-[4-(m-chlorophenyl)-1-piperazinyl]propyl]-s-triazolo[4,3-a]pyridine-3(2H)-one).

7. The therapeutic agent for Huntington's disease according to Claim 6, wherein the pharmaceutically acceptable salt of the compound represented by the formula (2-1) is a hydrochloride salt of the compound represented by the formula (2-2) (2-[3-[4-(m-chlorophenyl)-1-piperazinyl]propyl]-s-triazolo[4,3-a]pyridine-3(2H)-one hydrochloride).

8. The therapeutic agent for Huntington's disease according to Claim 1 or 2, wherein the compound represented by the formula (3-1) is a compound represented by the following formula (3-2): 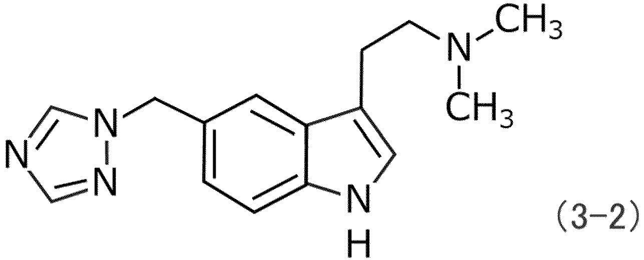 (N,N-dimethyl-2-[5-(1H-1,2,4-triazole-1-ylmethyl)-1H-indole-3-yl]ethaneamine).

9. The therapeutic agent for Huntington's disease according to Claim 8, wherein the pharmaceutically acceptable salt of the compound represented by the formula (3-1) is a benzoate of the compound represented by the formula (3-2) (N,N-dimethyl-2-[5-(1H-1,2,4-triazole-1-ylmethyl)-1H-indole-3-yl]ethaneamine benzoate).

10. The therapeutic agent for Huntington's disease according to Claim 1 or 2, wherein the compound represented by the formula (4-1) is a compound represented by the following formula (4-2): 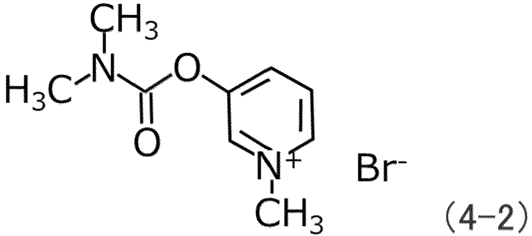 (3-dimethylcarbamoyloxy-1-methylpyridinium bromide).

11. A pharmaceutical composition for treating Huntington's disease comprising:
a compound represented by the following formula (1-1): 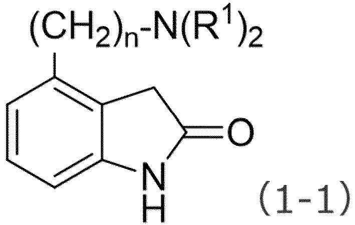 [wherein, in the formula (1-1), R¹ independently represents an alkyl group having 1 to 6 carbon atoms or a 4-hydroxyphenethyl group, and n represents an integer from 1 to 3],
a compound represented by the following formula (2-1): 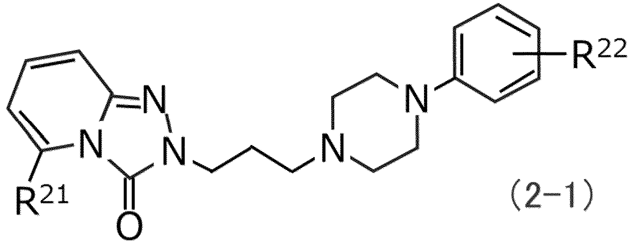
[wherein, in the formula (2-1), R²¹ is H or -CH₃,
R²² is selected from the group consisting of H, an alkyl group having 1 to 6 carbon atoms, an alkoxy group having 1 to 6 carbon atoms, and a halogen],
a compound represented by the following formula (3-1): 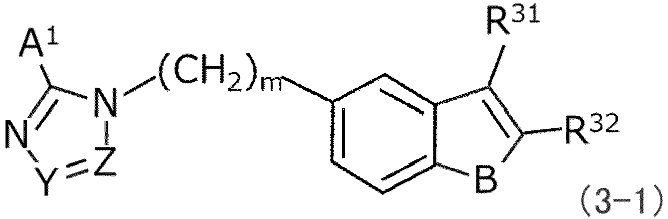
[wherein, in the formula (3-1),
Y represents N or A²-C,
Z represents N or CH,
m is 0, 1, 2 or 3,
B represents O, S or N-R³³,
A1 and A2 independently represent an optionally substituted alkyl group having 1 to 6 carbon atoms, an optionally substituted alkenyl group having 2 to 6 carbon atoms, an optionally substituted alkynyl group having 2 to 6 carbon atoms, an optionally substituted cycloalkyl group having 3 to 7 carbon atoms, an optionally substituted aryl group, an optionally substituted aryl-alkyl group having 1 to 6 carbon atoms, an optionally substituted heterocycloalkyl group having 3 to 7 carbon atoms, an optionally substituted heteroaryl group, or an optionally substituted heteroaryl-alkyl group having 1 to 6 carbon atoms, or H, halogen, cyano, trifluoromethyl, an alkoxy group having 1 to 6 carbon atoms, an alkylthio group having 1 to 6 carbon atoms, or -NR^{x}R^{y},
wherein R^{x} and R^{y} independently represent H, a hydrocarbon group, or a heterocyclic group, or R^{x} and R^{y} together represent an alkylene group having 2 to 6 carbon atoms,
R³¹ represents -CH₂CHR³⁴NR³⁵R³⁶ or the following formula: 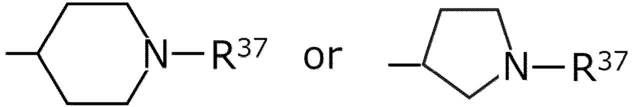
R³², R³³, R³⁴, R³⁵, R³⁶, and R³⁷ independently represent H or an alkyl group having 1 to 6 carbon atoms],
a compound represented by the following formula (4-1): 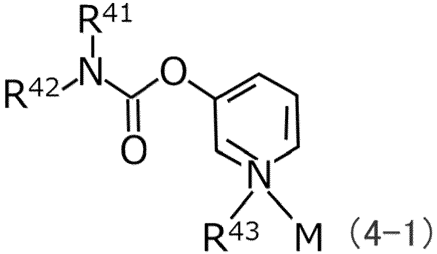
[wherein in the formula (4-1),
R⁴¹ and R⁴² are selected from an alkyl group having 1 to 6 carbon atoms, an aryl group having 6 to 10 carbon atoms, and an alkyl group having 1 to 6 carbon atoms substituted with an aryl group having 6 to 10 carbon atoms,
R⁴³ is selected from an alkyl group having 1 to 6 carbon atoms,
M is selected from halogens and alkyl sulfates],
or a pharmaceutically acceptable salt thereof, or a solvate thereof, as an active ingredient.
